# EUROPEAN PATENT APPLICATION

(11) **EP 2 390 322 A1**
(43) Date of publication of application: **30.11.2011**
(21) Application number: 10015711.4
(22) Date of filing: 16.12.2010
(51) Int. Cl.: C12N 9/64, C12N 15/10

(54) **Method for producing recombinant stem bromelain**

(30) Priority: 17.12.2009 MY 0905434
(71) Applicant: International Islamic University Malaysia, (IIUM), 50728 Kuala Lumpur (MY)
(72) Inventor: Amid, Azura, 53100 Kuala Lumpur (MY); Yusof, Faridah, 53100 Kuala Lumpur (MY); Ismail, Nurul Azira, 53100 Kuala Lumpur (MY)
(74) Representative: O'Connell, Maura

(57) **Abstract**

The present invention relates to a method for producing recombinant stem bromelain. Total RNA is isolated from pineapple plant (11) and used for reverse transcription polymerase chain reaction to generate multiple copies of stem bromelain gene (12). The gene is then cloned into a cloning vector (13), then transformed to a competent cell to produce an entry clone (14). After identifying and selecting the positively transformed entry clone (15), the stem bromelain gene is sub-cloned into a destination vector (16) and transformed into host cells to produce recombinant cells (17). After identifying and selecting the positively transformed recombinant cells (18), the recombinant cells are induced to express recombinant stem bromelain (19). Subsequently, the recombinant cells are harvested and the recombinant stem bromelain purified (20). The recombinant stem bromelain produced is in an active form and demonstrates activity similar to native stem bromelain.

SEQUENCE LISTING

<110>  INTERNATIONAL ISLAMIC UNIVERSITY MALAYSIA

<120>  METHOD FOR PRODUCING RECOMBINANT STEM BROMELAIN

<130>  PTA03.10

<150>  MY PI 20095434

<151>  2009-12-17

<160>  5

<210>  1
<211>  1839
<212>  DNA
<213>  Ananas comosus

<220>
<221>  CDS
<222>  (57)...(1112)

<400>  1
gtggctcaat ctccaagttc ctcatacata tactagatat ttagtgctac ataagc atg   59
                                                              Met
                                                               1

gct tcc aaa gtt caa ctc gtg ttt ctt ttc ttg ttt ctc tgt gcg atg   107
Ala Ser Lys Val Gln Lys Val Phe Leu Phe Leu Phe Leu Cys Ala Met
            5                   10                  15

tgg gct tcg cca tcg gca gct tct cgt gac gaa ccc aat gat ccc atg   155
Trp Ala Ser Pro Ser Ala Ala Ser Arg Asp Glu Pro Asn Asp Pro Met
        20                  25                  30

atg aag cgg ttt gaa gaa tgg atg gcg gag tac ggc cga gtt tac aag   203
Met Lys Arg Phe Glu Glu Trp Met Ala Glu Tyr Gly Arg Val Tyr Lys
    35                  40                  45

gac gac gac gag aag atg cgc cgg ttt cag ata ttc aag aac aac gtg   251
Asp Asp Asp Glu Lys Met Arg Arg Phe Gln Ile Phe Lys Asn Asn Val

 50                  55                  60                  65

aag cat atc gaa acc ttt aac agt cgc aac gaa aat tcg tac act ctc     299
Lys His Ile Glu Thr Phe Asn Ser Arg Asn Glu Asn Ser Tyr Thr Leu
                70                  75                  80

ggt atc aat cag ttt acc gat atg aca aaa agc gaa ttt gtt gct caa     347
Gly Ile Asn Gln Phe Thr Asp Met Thr Lys Ser Glu Phe Val Ala Gln
            85                  90                  95

tat acc ggc gta tct ctc cca cta aat atc gag aga gag cca gtg gtg     395
Tyr Thr Gly Val Ser Leu Pro Leu Asn Ile Glu Arg Glu Pro Val Val
        100                 105                 110

tca ttt gat gac gta aac atc tcc gcg gtg cct caa agt att gat tgg     443
Ser Phe Asp Asp Val Asn Ile Ser Ala Val Pro Gln Ser Ile Asn Trp
    115                 120                 125

aga gac tat ggt gcc gta aac gag gtc aag aat caa aac ccc tgt ggt     491
Arg Asp Tyr Gly Ala Val Asn Glu Val Lys Asn Gln Asn Pro Cys Gly
130                 135                 140                 145

tct tgc tgg tca ttc gct gca att gcg acg gtg gag gga atc tac aag     539
Ser Cys Trp Ser Phe Ala Ala Ile Ala Thr Val Glu Gly Ile Tyr Lys
                150                 155                 160

atc aaa aca ggg tac tta gta tct tta tcg gag caa gaa gtt ctc gat     587
Ile Lys Thr Gly Tyr Leu Val Ser Leu Ser Glu Gln Glu Val Leu Asp
            165                 170                 175

tgt gct gtt agc tac ggg tgc aaa ggc ggc tgg gtg aac aag gcc tac     635
Cys Ala Val Ser Tyr Gly Cys Lys Gly Gly Trp Val Asn Lys Ala Tyr
        180                 185                 190

gat ttc atc ata tct aac aac ggt gtg acc acc gaa gaa aac tat cct     683
Asp Phe Ile Ile Ser Asn Asn Gly Val Thr Thr Glu Glu Asn Tyr Pro
    195                 200                 205

tat cta gca tac caa ggc act tgc aac gcc aat agc ttt cct aat tca     731
Tyr Leu Ala Tyr Gln Gly Thr Cys Asn Ala Asn Ser Phe Pro Asn Ser
210                 215                 220                 225

gct tac att act ggt tat tca tat gtg cga agg aac gac gaa cgc agc     779
Ala Tyr Ile Thr Glu Tyr Ser Tyr Val Arg Arg Asn Asp Glu Arg Ser
                230                 235                 240

atg atg tac gct gtg tcg aat caa cca ata gct gct ctt atc gat gcc     827
Met Met Tyr Ala Val Ser Asn Gln Pro Ile Ala Ala Leu Ile Asp Ala
            245                 250                 255

agt gaa aac ttt caa tat tac aat ggc ggt gtg ttt agc gga cct tgt     875

Ser Glu Asn Phe Gln Tyr Tyr Asn Gly Gly Val Phe Ser Gly Pro Cys
        260                 265                 270

gga act agt ctc aat cat gcc att acc att ata ggt tac ggg cag gat     923
Gly Thr Ser Leu Asn His Ala Ile Thr Ile Ile Gly Tyr Gly Gln Asp
    275                 280                 285

agc agt gga aca aaa tat tgg ata gta agg aac tcg tgg ggc agc tca     971
Ser Ser Gly Thr Lys Tyr Trp Ile Val Arg Asn Ser Trp Gly Ser Ser
290                 295                 300                 305

tgg ggt gag ggt gga tac gtc cgt atg gca aga ggt gtg tca tcg tca     1019
Trp Gly Glu Gly Gly Tyr Val Arg Met Ala Arg Gly Val Ser Ser Ser
                310                 315                 320

tct gga gta tgt gga atc gcc atg gct cct ctc ttt ccc act cta caa     1067
Ser Gly Val Cys Gly Ile Ala Met Ala Pro Leu Phe Pro Thr Leu Gln
            325                 330                 335

tca ggg gct aat gcc gaa gtt att aag atg gtt tct gaa act tga         1112
Ser Gly Ala Asn Ala Glu Val Ile Lys Met Val Ser Glu Thr
        340                 345                 350

agctacgtaa taagtcggaa gcaaatgatt tagcaacact gcagtttgct atcttatttg
1172

ctttacttac ataaataagg atgtttgctg catccaattg ctaagcttgc ttcgacattg
1232

tggtttctgt tctgtttcgg tgtgtcctgc cagtatgtaa gtttgaatat ctataattga   1292

atgtgctcct tcattattgt gtactaaaaa aaaaaaaaaa aaaaaaaaaa aaaaaaaaaa   1352

aaaaaaaaaa aaaaaaaaaa aggggggggg ctccccaccc tgctagggtt ttggagggcc   1412

cccattctcc tcctccgcac ctcgccccca accgccccct ctctctctgt aaccataacc   1472

ctagccctag ggaattaggg atctcgtcgt ctagggttta ggcggagctc gcgcgccatg   1532

gatagataca ctagagtgga gaagccgcgg ccggaatcgg tcataaacga gaacgagatc   1592

cgaatcacgt cgcagggact catccggaac tatgtgagct acgctacgag cctcctacag   1652

gaaaaacggg tgcgagagat tgttttgaag gccatgggac aggcaatcag caaggctgtg   1712

gctattgcag agataataaa gaaaaggaac cctgggttgc atcaagatac ctctatcagt   1772

tctgtcagta tcactgatgt gtgggagcct gttgaagagg gccttgtacc attggaattg   1832

acacgcc                                                             1839

<210>  2

<211>  351
<212>  PRT
<213>  Ananas comosus

<400>  2

Met Ala Ser Lys Val Gln Lys Val Phe Leu Phe Leu Phe Leu Cys Ala
1               5                   10                  15

Met Trp Ala Ser Pro Ser Ala Ala Ser Arg Asp Glu Pro Asn Asp Pro
            20                  25                  30

Met Met Lys Arg Phe Glu Glu Trp Met Ala Glu Tyr Gly Arg Val Tyr
        35                  40                  45

Lys Asp Asp Asp Glu Lys Met Arg Arg Phe Gln Ile Phe Lys Asn Asn

        50                  55                  60

Val Lys His Ile Glu Thr Phe Asn Ser Arg Asn Glu Asn Ser Tyr Thr
65                  70                  75                  80

Leu Gly Ile Asn Gln Phe Thr Asp Met Thr Lys Ser Glu Phe Val Ala
                85                  90                  95

Gln Tyr Thr Gly Val Ser Leu Pro Leu Asn Ile Glu Arg Glu Pro Val
            100                 105                 110

Val Ser Phe Asp Asp Val Asn Ile Ser Ala Val Pro Gln Ser Ile Asn
        115                 120                 125

Trp Arg Asp Tyr Gly Ala Val Asn Glu Val Lys Asn Gln Asn Pro Cys
    130                 135                 140

Gly Ser Cys Trp Ser Phe Ala Ala Ile Ala Thr Val Glu Gly Ile Tyr
145                 150                 155                 160

Lys Ile Lys Thr Gly Tyr Leu Val Ser Leu Ser Glu Gln Glu Val Leu
                165                 170                 175

Asp Cys Ala Val Ser Tyr Gly Cys Lys Gly Gly Trp Val Asn Lys Ala
            180                 185                 190

Tyr Asp Phe Ile Ile Ser Asn Asn Gly Val Thr Thr Glu Glu Asn Tyr
        195                 200                 205

Pro Tyr Leu Ala Tyr Gln Gly Thr Cys Asn Ala Asn Ser Phe Pro Asn
    210                 215                 220

Ser Ala Tyr Ile Thr Glu Tyr Ser Tyr Val Arg Arg Asn Asp Glu Arg
225                 230                 235                 240

Ser Met Met Tyr Ala Val Ser Asn Gln Pro Ile Ala Ala Leu Ile Asp
                245                 250                 255

Ala Ser Glu Asn Phe Gln Tyr Tyr Asn Gly Gly Val Phe Ser Gly Pro
            260                 265                 270

Cys Gly Thr Ser Leu Asn His Ala Ile Thr Ile Ile Gly Tyr Gly Gln
        275                 280                 285

Asp Ser Ser Gly Thr Lys Tyr Trp Ile Val Arg Asn Ser Trp Gly Ser
    290                 295                 300

Ser Trp Gly Glu Gly Gly Tyr Val Arg Met Ala Arg Gly Val Ser Ser
305                 310                 315                 320

Ser Ser Gly Val Cys Gly Ile Ala Met Ala Pro Leu Phe Pro Thr Leu
                325                 330                 335

Gln Ser Gly Ala Asn Ala Glu Val Ile Lys Met Val Ser Glu Thr
            340                 345                 350

<210>  3
<211>  16
<212>  DNA
<213>  Artificial Sequence
<220>
<223>
<400>  3
at ggc ttc caa agt tc                                              16

<210>  4
<211>  22
<212>  DNA
<213>  Artificial Sequence
<220>
<223>
<400>  4
cta aat cat ttg ctt ccg act t                                      22

<210>  5
<211>  21
<212>  DNA
<213>  Artificial Sequence
<220>
<223>
<400>  5
cta aat cat ttg ctt cga ctt                                        21

## Description

### Field of the Invention

This invention relates to a method for producing a recombinant protein, and more particularly to a method for producing recombinant stem bromelain.

### Description of Related Arts

Commercial bromelain is a crude, aqueous extract from the stems and immature fruits of pineapples. Crude pineapple stem preparation contains two distinct cysteine proteases: stem bromelain and ananain. Three cysteine proteases are present in the pineapple stem: stem bromelain, ananain, and comosain. Bromelain is a protein-digesting enzyme similar in function to papain and ficin. It is also the most cost effective natural protease.

Bromelain is known to have a number of uses. A known pharmaceutical application of bromelain is the enzymic debridement of necrotic tissues from ulcers and burn wounds. Other therapeutic benefits include reversible inhibition of platelet aggregation, management of bronchitis, improved healing of surgical traumas and enhancement of drug absorption, particularly of antibiotics. It has also been reported to have anticancer properties. A major commercial use of bromelain is in food processing, especially with meat tenderizing processes. Approximately 95 percent of the meats tenderizing enzymes consumed in the United States are from the plant proteases, which are papain and bromelain. Bromelain is utilized because its ideal temperature range is ~ 49 - 71°C (Rojek, 2003) which is suitable for industrial application

For commercialization, bromelain is prepared from cooled pineapple juice by centrifugation, ultra filtration, and lyophilization. However, this extract mainly comprises glycosylated multiple enzyme species of the papain super family with different proteolytic activities and with molecular masses ranging between 20 to 31 kDa, and isoelectric points ranging between 10 to 4.8 (Harrach et al., 1995). Many approaches have been applied to increase the purity and activity of this enzyme, and some have applied liquid-liquid extraction procedures during the extraction-preparation process (Ravindra Babu *et al.,* 2008). Others approached it at the purification stage by precipitation and chromatography techniques, followed by a freeze-drying step to increase the purity and activity of the stem bromelain (Devakate *et al.* 2009). Unfortunately, all these attempts require high cost of materials and operation. It is, therefore, highly desirable to find alternative method of producing highly purified bromelain.

### Summary of Invention

It is an object of the present invention to provide a method for producing highly purified stem bromelain.

It is also an object of the present invention to provide a cost effective method for producing high amounts of stem bromelain.

It is a further object of the present invention to provide a method for producing stem bromelain using a genetic recombination method.

Accordingly, these objectives may be achieved by following the teachings of the present invention. The present invention relates to a method for producing recombinant stem bromelain comprising the steps of: firstly, isolating total RNA from pineapple plant material (11); then, performing reverse transcription polymerase chain reaction on the RNA to generate multiple copies of stem bromelain gene (12); then, cloning the stem bromelain gene into cloning vectors (13); then, transforming the cloning vector into a competent cell to produce an entry clone (14); then, identifying and selecting the positively transformed entry clone (15); then, subcloning the stem bromelain gene from the entry clone into a destination vector (16); then, transforming the destination vector into a host cell to produce recombinant cells (17); then, identifying and selecting the positively transformed recombinant cells (18); then, inducing the recombinant cells to express recombinant stem bromelain (19); and subsequently, harvesting the recombinant cells and purifying the recombinant stem bromelain (20); wherein the recombinant stem bromelain produced is in an active form and demonstrates activity similar to native stem bromelain.

### Brief Description of the Drawings and Sequence Listing

The features of the invention will be more readily understood and appreciated from the following detailed description when read in conjunction with the accompanying drawing, in which:
Fig. 1 is a flow chart of the method for producing recombinant stem bromelain.
Fig. 2 is a sequence of the stem bromelain gene (D14059.1) from *Ananas comosus* obtained from the National Centre of Biotechnology Information (NCBI).
Fig. 3a is an SDS-PAGE result for restriction enzyme digestion of positively transformed entry clones by *Cla*I and *Not*I
Fig. 3b is an SDS-PAGE result for restriction enzyme digestion of positively transformed recombinant cells by *Aat*II and *Spe*I.
Figure 4 is an SDS-PAGE result for different samples of the recombinant stem bromelain from different purification steps performed under denatured conditions.
Fig. 5a is an SDS-PAGE analysis of a sample of the recombinant stem bromelain.
Fig. 5b is a Western blot analysis of the same sample of the recombinant stem bromelain as used in Fig. 5a.
Fig. 6 is a graph of average cycle threshold (Ct) values obtained from real-time PCR studies of bromelain expression among different colonies of recombinant *E. coli* harboring heterologous bromelain, with and without L-arabinose induction.
Fig. 7 is a graph of recombinant stem bromelain activity against Nα-CBZ-L-Lysine p-nitrophenyl ester, LNPE, at different pH levels.
Fig. 8 is a graph of recombinant stem bromelain activity against Nα-CBZ-L-Lysine *p*-nitrophenyl ester, LNPE, at different temperatures.

In the Sequence Listing, there is provided a nucleotide sequence of the stem bromelain gene (D14059.1) (SEQ ID NO.1) from *Ananas comosus* and its corresponding amino acid sequence; the amino acid sequence of stem bromelain (SEQ ID NO.2) from *Ananas comosus* encoded by the sequence of SEQ ID NO. 1; a nucleotide sequence of a forward primer BroM-F (SEQ ID NO. 3); a reverse primer BroM-R sequence (SEQ ID NO.4) and another reverse primer BroM-R sequence (SEQ ID NO. 5).

### Detailed Description of the Invention

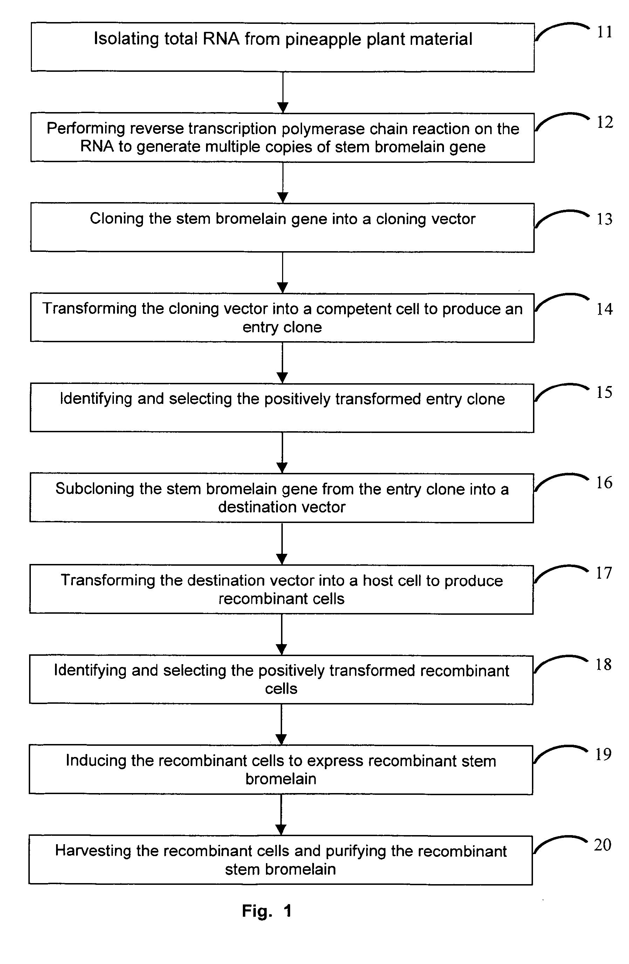

As required, detailed embodiments of the present invention are disclosed herein; however, it is to be understood that the disclosed embodiments are merely exemplary of the invention, which may be embodied in various forms. Therefore, specific structural and functional details disclosed herein are not to be interpreted as limiting but merely as a basis for claims. It should be understood that the drawings and detailed description thereto are not intended to limit the invention to the particular form disclosed, but on the contrary, the invention is to cover all modification, equivalents and alternatives falling within the spirit and scope of the present invention as defined by the appended claims. As used throughout this application, the word "may" is used in a permissive sense (i.e., meaning having the potential to), rather than the mandatory sense (i.e., meaning must). Similarly, the words "include," "including," and "includes" mean including, but not limited to. Further, the words "a" or "an" mean "at least one" and the word "plurality" means one or more, unless otherwise mentioned. Where the abbreviations of technical terms are used, these indicate the commonly accepted meanings as known in the art. For ease of reference, common reference numerals will be used throughout the figures when referring to the same or similar features common to the figures. The present invention will now be described with reference to Figs. 1-8.

As shown in Fig. 1, the present invention relates to a method for producing recombinant stem bromelain comprising the steps of: firstly, isolating total RNA from pineapple plant material (11); then, performing reverse transcription polymerase chain reaction on the RNA to generate multiple copies of stem bromelain gene (12); then, cloning the stem bromelain gene into cloning vectors (13); then, transforming the cloning vector into a competent cell to produce an entry clone (14); then, identifying and selecting the positively transformed entry clone (15); then, subcloning the stem bromelain gene from the entry clone into a destination vector (16); then, transforming the destination vector into a host cell to produce recombinant cells (17); then, identifying and selecting the positively transformed recombinant cells (18); then, inducing the recombinant cells to express recombinant stem bromelain (19); and subsequently, harvesting the recombinant cells and purifying the recombinant stem bromelain (20); wherein the recombinant stem bromelain produced is in an active form and demonstrates activity similar to native stem bromelain.

Firstly, the total RNA from pineapple plant material must be isolated from pineapple plant material (11). Preferably, the total RNA is isolated from the stems of the pineapple plant as the stems have the highest concentration of bromelain and are easily available after the fruit is harvested. Any method for RNA isolation known in the art may be used, for example, column-based nucleic acid purification, ethanol precipitation or phenol-chloroform extraction. Consideration must be given to the storage and handling of the fresh pineapple plant material. Preferably, the pineapple plant material is treated with a stabilizing agent, flash-frozen, or disrupted and homogenized in the presence of RNase-inhibiting or denaturing reagents after harvesting to prevent unwanted changes in the gene expression profile from occurring. For example, tissue samples should be immediately frozen in liquid nitrogen and stored at -70°C.

To begin isolating the total RNA from pineapple plant material (11), the pineapple plant material should first be disrupted, then homogenized. Disruption involves the complete disruption of cell walls and membranes of cells and organelles to release the total RNA contained in the pineapple plant material sample. Any device capable of thoroughly grinding the pineapple plant material may be used, e.g. mortar and pestle. Typically, the pineapple plant material is frozen immediately in liquid nitrogen and ground to a fine powder under liquid nitrogen. The suspension of tissue powder and liquid nitrogen is then transferred into a liquid-nitrogen-cooled, appropriately sized tube, wherein the liquid nitrogen is allowed to evaporate without allowing the sample to thaw. Then, a lysis buffer is added to the disrupted pineapple plant material.

Next, the disrupted pineapple plant material is homogenized using a homogenizer. Homogenization should be carried out as quickly as possible after the disruption of the pineapple plant material. The purpose of homogenization is to reduce the viscosity of the lysate sample produced by disruption. Homogenization helps to shear high-molecular-weight genomic DNA and other high-molecular-weight cellular components in the lysate sample, thereby creating a homogeneous lysate. Incomplete homogenization may result in significantly reduced RNA yields. After homogenization, the lysate sample may then be treated according to common protocols of RNA isolation from plant material, depending on the RNA isolation method chosen.

Once the total RNA from pineapple plant material has been isolated from pineapple plant material (11), this is followed by performing reverse transcription polymerase chain reaction on the RNA to generate multiple copies of stem bromelain gene (12). Preferably, this comprises the steps of: firstly, reverse transcribing the RNA to cDNA using a forward primer BroM-F (5'-AT GGC TTC CAA AGT TC-3') (shown in SEQ ID NO. 3) and a reverse primer BroM-R selected from a group consisting of a sequence (5'- CTA AAT CAT TTG CTT CCG ACT T -3') shown in SEQ ID NO. 4, and a sequence (5'- CTA AAT CAT TTG CTT CGA CTT -3') shown in SEQ ID NO. 5, then amplifying the cDNA by polymerase chain reaction to generate multiple copies of the stem bromelain gene. The forward primer BroM-F and reverse primer BroM-R may be designed using a primer design software and synthesized prior to the reverse transcription step. The forward primer BroM-F may further include additional base pair sequences at the 5' end to ensure compatibility of the forward primer BroM-F with the cloning vector.

For reverse transcription, reagents comprising buffer, dNTP mix, the forward primer BroM-F and reverse primer BroM-R, reverse transcriptase and RNase inhibitor are added into a microcentrifuge tube, which is then incubated. The reaction can be terminated by heating the reaction mixture to about 70°C. The cDNA thus produced is then amplified by polymerase chain reaction.

The reagents for polymerase chain reaction typically comprise of buffer, dNTP mix, the forward primer BroM-F and reverse primer BroM-R, the cDNA and *Taq* DNA polymerase. The polymerase chain reaction for amplifying the cDNA is preferably performed for 30 cycles at 94°C (2 minutes) for denaturing, 58°C (30 seconds) for annealing and 72°C (2 minutes) for extension. Additionally, the copies of stem bromelain gene generated by the reverse transcription polymerase chain reaction may then be sequenced and blasted against a gene database, e.g. the National Center for Biotechnology Information (NCBI) database, to confirm the identity of said stem bromelain gene. This helps to confirm that the correct gene was amplified. Successful reverse transcription polymerase chain reaction will result in multiple copies of stem bromelain gene being generated.

The next step is cloning the stem bromelain gene into cloning vectors (13). Although various types of vectors are known in the art, the preferred cloning vector of the present invention is a plasmid. The cloning of the stem bromelain gene into cloning vectors (13) may be performed by incubating the stem bromelain gene with reagents comprising salt solution, sterile water and the cloning vector. Then, the mixture is placed on ice before proceeding to the next step.

After cloning the stem bromelain gene into cloning vectors (13), the cloning vector is transformed into a competent cell to produce an entry clone (14). This step preferably comprises of incubating the cloning vector with a naturally or artificially competent bacterial cell until the stem bromelain gene carried by the cloning vector has transfected the competent cell and integrated with the genome of the competent cell. In the preferred embodiment, the competent cell is *Escherichia coli,* including variants, strains and recombinants thereof. Although various methods are known in the art to induce artificial competence in a cell, the most common are chemical methods or electroporation. In the present invention, chemically competent cells are preferred. Heat shock treatment may also be applied during the transformation process to induce competence in said cells.

Next, the positively transformed entry clone is identified and selected (15), preferably by colony polymerase chain reaction and restriction enzyme digestion by *Cla*I and *Not*I*. Cla*I recognizes the restriction site 5'-ATCGAT-3' while *Not*I recognizes the restriction site 5'-GCGGCCGC-3'.

Then, the stem bromelain gene from the entry clone is subcloned into a destination vector (16). In the preferred embodiment, the destination vector is a plasmid that can express the stem bromelain gene in a host cell. Preferably, the subcloning of the stem bromelain gene from the entry clone into the destination vector (16) is by LR recombination. LR recombination is an example of site-specific recombination. To subclone the stem bromelain gene from the entry clone into a destination vector (16), the entry clone may be incubated with the destination vector in the presence of enzymes and buffer. For LR recombination, the enzymes comprise of integrase (Int), excisionase (Xis) and Integration Host Factor (IHF) (1).

Following this is the step of transforming the destination vector into a host cell to produce recombinant cells (17). This may be done by incubating the destination vector with a naturally or artificially competent bacterial host cell until the stem bromelain gene carried by the destination vector has transfected the host cell and integrated with the genome of the host cell. Preferably, the host cell is *Escherichia coli,* including variants, strains and recombinants thereof. Heat shock treatment may also be applied during the transformation process to induce competence in the host cell.

After that, the positively transformed recombinant cells are identified and selected (18), preferably by colony polymerase chain reaction and restriction enzyme digestion by *Aat*II and *Spe*I. *Aat*II recognizes the restriction site 5'-GACGTC-3' while Spel recognizes the restriction site 5'-ACTAGT-3'.

The next step is inducing the recombinant cells to express recombinant stem bromelain (19), which preferably comprises the steps of: firstly, subculturing the positively transformed recombinant cells in a cell culture medium until the mid-logarithmic phase of cell growth; then, adding L-arabinose to the recombinant cells to induce expression of the recombinant stem bromelain; and subsequently, incubating the recombinant cells with L-arabinose. Preferably, the recombinant cells are incubated with L-arabinose at temperature of 37°C and agitation of 200 rpm for three hours.

Subsequently, the recombinant cells are harvested and the recombinant stem bromelain is purified (20). In the preferred embodiment, this comprises the steps of: firstly, harvesting the recombinant cells; then, centrifuging the recombinant cells; then, lysing the recombinant cells to produce a lysate; then, centrifuging the lysate to collect a supernatant; and subsequently, extracting the recombinant stem bromelain from the supernatant. Extraction of the recombinant stem bromelain from the supernatant may be done using various methods, depending on the nature of the recombinant stem bromelain expressed. For instance, if the recombinant stem bromelain is expressed with N-terminal polyhistidine-tag (6xHis tag), a nickel-chelating resin such as Ni-NTA (nickel-nitrilotriacetic acid) can be used to extract the recombinant stem bromelain.

The following non-limiting examples are provided to illustrate the present invention and in no way limits the scope thereof.

### Example

### Method

About 200 mg fresh maturing pineapple stems (from *Ananas comosus* N36) were used for RNA isolation. The pineapple stems were weighed and placed in liquid nitrogen, then ground with mortar and pestle to produce a tissue powder. The tissue powder and liquid nitrogen were then decanted into an RNase-free, liquid-nitrogen-cooled, microcentrifuge tube. Once the liquid nitrogen had evaporated, a lysis buffer containing guanidine thiocyanate was immediately added, and the tissue and buffer mixture was vortexed, producing a lysate. Next, the lysate was transferred to a homogenizer and centrifuged for 2 min to remove insoluble plant material, homogenize the lysate and reduce lysate viscosity. The supernatant was collected and transferred to a new microcentrifuge tube before adding 0.5 volume of ethanol (96-100%) to the cleared lysate to create conditions and mixing thoroughly to promote selective binding of RNA to the spin column membrane used for extracting the RNA.

The lysate (about 650 µl) and any precipitate formed were transferred to the spin column and centrifuged for 15 s at ≥8000 x g (≥10,000 rpm). The flow-through was discarded. To wash the lysate sample, buffer solution was added to the spin column then centrifugation was performed again at ≥8000 x g (≥10,000 rpm) and the flow-through discarded. This was repeated twice more. Then, 30-50 µl RNase-free water was added to the spin column membrane and centrifugation performed for 1 minute at ≥8000 x g (≥10,000 rpm). The elute, which contained RNA, was then collected.

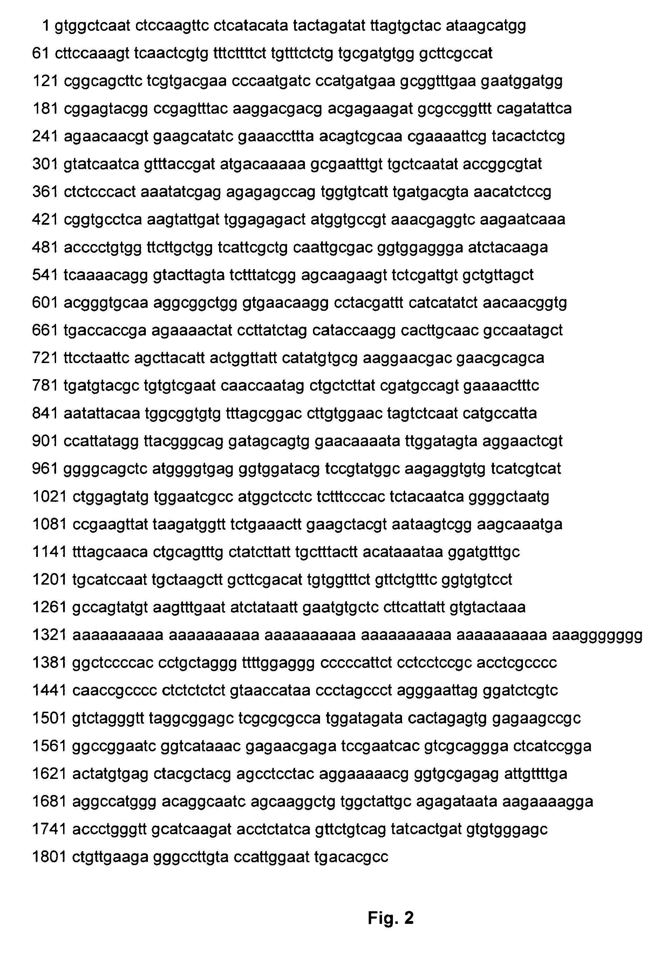

Next, the total RNA is amplified by reverse transcription polymerase chain reaction. The complete mRNA sequence of the stem bromelain gene (D14059.1), as shown in Fig. 2 and SEQ ID NO. 1, was accessed from the National Centre of Biotechnology Information (NCBI). The corresponding amino acid sequence is shown in SEQ ID NO. 2. A pair of primers BroM-F (5'-CAC CAT GGC TTC CAA AGT TC-3') (SEQ ID NO. 3) and BroM-R (selected from a group consisting of a sequence (5'- CTA AAT CAT TTG CTT CCG ACT T -3') SEQ ID NO. 4, and a sequence (5'- CTA AAT CAT TTG CTT CGA CTT -3') SEQ ID NO. 5) were designed using a primer design software, Primer3 version 4.0 and synthesized to be used for reverse transcription polymerase chain reaction experiments. The CCAC bases were added to the forward primer in order to make it compatible with the TOPO® cloning vector (Invitrogen, USA). The total RNA isolated from pineapple stems was first reverse transcribed to cDNA. The primers (about 2 pmol), total RNA (5 µg) and 1µl of 10 mM pH neutral dNTP mix were added into a nuclease-free microcentrifuge tube with sterile distilled water. The mixture was heated to 65°C for 5 minutes, then incubated on ice for at least 1 minute, then briefly centrifuged. Buffer solution (250 mM Tris-HCl (pH 8.3 at room temperature), 375 mM KCI, 15 mM MgCl₂), dithiothreitol, ribonuclease inhibitor and reverse transcriptase were then added to the tube and mixed. The mixture was incubated at 55°C for 30-60 minutes. The reaction was inactivated by heating the mixture up to 70°C for 15 minutes. The obtained cDNA was used as a template for PCR. PCR buffer (200 mM Tris-HCl (pH 8.4), 500 mM KCI), MgCl₂, dNTP mix, primers, *Taq* DNA polymerase, cDNA and sterile water are mixed together for the PCR reaction. PCR reaction was performed for 30 cycles at 94°C (2 minutes) for denaturing, 58°C (30 seconds) for annealing and 72°C (2 minutes) for extension. The PCR product was sequenced and the sequencing results were blasted against the NCBI database to confirm that the right gene was amplified.

Subsequently, this PCR product was cloned into the pENTR/TEV/D-TOPO® cloning vector (Invitrogen, USA). The molar ratio of PCR product:TOPO® vector used should be from 0.5:1 to 2:1. Fresh PCR product, salt solution (1.2 M NaCl and 0.06 M MgCl₂), water, TOPO® vector were mixed together and incubated for 5 minutes at room temperature. Then the mixture was placed on ice to terminate the reaction. The mixture was then immediately transformed into chemically competent *E*. *coli.* In this study, One Shot® TOP10 Chemically Competent *E*. *coli* cells (Invitrogen, USA) were used. The mixture from the cloning reaction was mixed with the chemically competent *E*. *coli* and incubated on ice for up to 30 minutes. Then, the cells were heat-shocked for 30 seconds at 42°C without shaking. Immediately, the mixture was placed on ice and S.O.C. medium (2% w/v Tryptone, 0.5% w/v Yeast Extract, 10 mM NaCl, 2.5 mM KCI, 10 mM MgCl₂, 10 mM MgSO₄, 20 mM glucose) was added to form a culture before incubating at 37°C for 1 hour with shaking. The culture was then spread on a selective plate and incubated overnight at 37°C.

Positively transformed entry clones were selected by colony PCR and restriction enzyme digestion. For colony PCR, a sample of 1 µL of each colony was diluted with distilled water with a ratio 1:10. Then, the PCR master mix was prepared using Go Taq® Flexi DNA Polymerase (Promega, USA) according to manufacturer's instructions. 2 µL of the colony mixture was mixed with 1x reaction buffer, 2.5mM of MgCl₂, 0.4 pM of primer, 0.2 mM of dNTP and 1.25U/µL of Go Taq® DNA Polymerase. The total volume of the reaction was 20 µL. Then, the size of the transformants was confirmed by agarose gel electrophoresis.

The purified plasmid was double digested with two restriction enzymes *Cla*I and *Not*I (NEB, USA) according to table below. Since the double digestion required two different types of restriction enzymes, the buffer to be applied in the master mix reaction should be applicable to both of the enzymes. The reagents used are listed in Table 1 below. The result was studied by agarose gel eletrophoresis to see the bands that might be produced from the reaction.

**Table 1: Reagents for restriction enzyme digestion by ClaI and NotI**

| **Reagents** | **Volume (µL)** |
|---|---|
| Buffer | 2 |
| (1X NEBuffer 3: | |
| 100 mM NaCl | |
| 50 mM Tris-HCl | |
| 10 mM MgCl2 | |
| 1 mM Dithiothreitol | |
| pH 7.9 at 25°C) | |
| Bovine Serum Albumin (BSA) | 0.2 |
| Restriction Enzyme 1 (*Cla*I) | 1 |
| Restriction Enzyme 2 (*Not*I) | 1 |
| Purified Plasmid | 5 |
| Distilled Water | 10.8 |
| Total Volume | 20 |

Next, the stem bromelain gene was subcloned from the entry clone into the destination vector by LR recombination reaction. The destination vector used in this experiment was the pDEST^{™}17 vector (Invitrogen, USA). This was done by incubating the entry clone with the pDEST^{™}17 vector in the presence of TE buffer (at pH 8.0) and enzyme mix (Integrase (Int), Excisionase (Xis), Integration Host Factor (IHF) (1), and reaction buffer) at 25°C for 1 hour. Proteinase solution (proteinase 2 µg/µl in 10 mM Tris-HCl, pH 7.5, 20 mM CaCl₂ and 50% glycerol) is then added to terminate the reaction. The mixture was then vortexed briefly before re-incubating at 37°C for 10 minutes.

The destination vector was then transformed into the cells (Invitrogen, USA), which are BL21-derived *E*. *coli* cells to produce recombinant cells. The BL21-Al^{™} host cells are gently mixed with the destination vector and incubated on ice for 30 minutes. Then the cells are heat-shocked for 30 seconds at 42°C without shaking. The mixture is then immediately transferred to ice. S.O.C. medium (room temperature) is then added to the mixture. The mixture was agitated (200 rpm) at 37°C for 30 minutes. The cells were then plated on a pre-warmed selective plate and incubated overnight at 37°C.

Positively transformed recombinant cells were identified and selected by colony PCR and restriction enzyme digestion as before. The restriction enzymes used were *Aat*II and *Spe*I. The reagents used for the restriction enzyme digestion are listed in Table 2.

**Table 2: Reagents for restriction enzyme digestion by AatII and SpeI**

| **Reagents** | **Volume (µL)** |
|---|---|
| Buffer | 2 |
| (1X NEBuffer 4: | |
| 50 mM potassium acetate | |
| 20 mM Tris-acetate | |
| 10 mM Magnesium Acetate | |
| 1 mM Dithiothreitol | |
| pH 7.9 at 25°C) | |
| Bovine Serum Albumin (BSA) | 0.2 |
| Restriction Enzyme 1 (*Aat*II) | 1 |
| Restriction Enzyme 2 (*Spe*I) | 1 |
| Purified Plasmid | 5 |
| Distilled Water | 10.8 |
| Total Volume | 20 |

Next, the recombinant cells were induced to express the recombinant stem bromelain. Firstly, the recombinant cells were cultured in 100 mL LB medium with agitation 200 rpm at 37°C until the cell culture reached the mid-log phase (OD₆₀₀ₙₘ = 0.4-0.6). Then, 0.2% L-arabinose was added to induce recombinant stem bromelain expression. The cultures continued to be incubated at 37°C with shaking. For sampling, a 500 µl aliquot was removed from the culture and centrifuged at maximum speed in a microcentrifuge for 30 seconds. The supernatant was aspirated and the cell pellets were frozen at-20°C. The frozen pellets were later thawed for analysis.

The cell was harvested three hours after L-arabinose induction and centrifuged at 5000 g, 15 minutes at 4°C. Induced *E*. *coli* was then lysed using buffer B-7M urea (7 M urea, 0.1 M NaH₂PO₄, 0.01 M Tris-Cl, pH 8.0) under denaturing and native conditions and purified using an Ni-NTA (nickel-nitrilotriacetic acid) spin column (Qiagen, Germany). The harvested cells were lysed using lysis buffer depending on the type of purification (denatured or native conditions). The purification of the protein was carried out at room temperature and 4°C for denatured and native conditions respectively. The lysate was centrifuged at 12,000 x g to collect the supernatant. The supernatant was applied to the equilibrated Ni-NTA (nickel-nitrilotriacetic acid) spin column. Ni-NTA (nickel-nitrilotriacetic acid) spin column was the purification method chosen because the pDEST^{™}17 destination vector used here has the N-terminal polyhistidine-tag (6xHis tag). The polyhistidine-tag on the recombinant stem bromelain would therefore attach to the Ni-NTA in the column. The column was washed with buffer to remove the unneeded proteins and substances. The recombinant bromelain was eluted at the end of the purification process.

### Results and discussion

### Recombinant bromelain clone

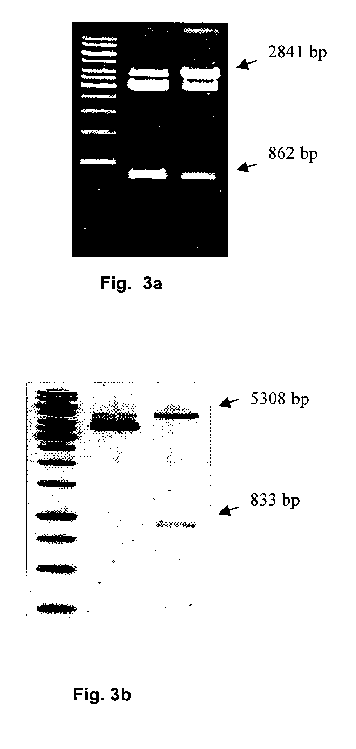

The blast results showed that the amplified RNA fragment had 98% similarity to the mRNA sequence of the stem bromelain gene (D14059.1) shown in Fig. 2 and SEQ ID NO. 1. This is possible because the NCBI mRNA sequence of the bromelain gene was from a different pineapple variety. The amplified fragments were successfully cloned into pENTR/TEV/D-TOPO® cloning vector (Invitrogen, USA). Restriction enzyme digestion by *Cla*I and *Not*I identified the positive clone having two expected fragments with 2841 and 862 bps in size (Fig. 3a). Plasmid from the positive clone was then successfully sub cloned into pDEST^{™}17 destination vector by LR recombination and restriction enzyme digestion by *Aat*II and *Spe*I identified the positive clone having two expected fragments of 5308 bp and 833 bp (Fig. 3b).

### Expression of recombinant stem bromelain

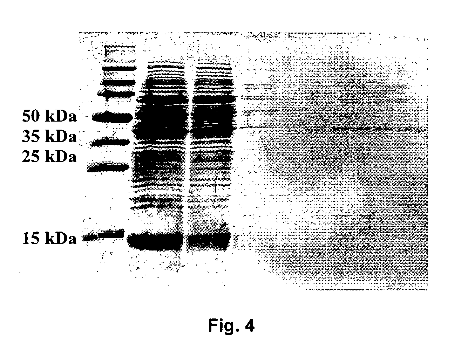

To express recombinant stem bromelain from the positively identified clone, recombinant E. *coli* culture, grown in LB-medium, was induced by 0.2 % of L-arabinose. Fig. 4 shows the SDS-PAGE result for different samples from different purification steps performed under denatured conditions. Lanes five and six in Fig. 4 show the elution of the purified protein off the Ni-NTA spin column. Only one band appeared on this lane, which suggested that this protein has the polyhistidine-tag attached and had successfully eluted from the column and this protein lies between 35 kDa and 50 kDa. The molecular weight of this heterologous bromelain is bigger than the expected size (28 kDa). The difference in size of the recombinant protein might be due to posttranslational modification. In this case, posttranslational modification occurred because bromelain is a protease originating from the pineapple, which is eukaryote. This protease was cloned into a prokaryotic expression system (*E*. *coli*)*. E.coli* is a well known expression system which can generate high levels of expression. But prokaryotes do not carry the same kinds of posttranslational modification as eukaryotes do. Posttranslational modification may affect activity, stability or folding pattern of a protein.

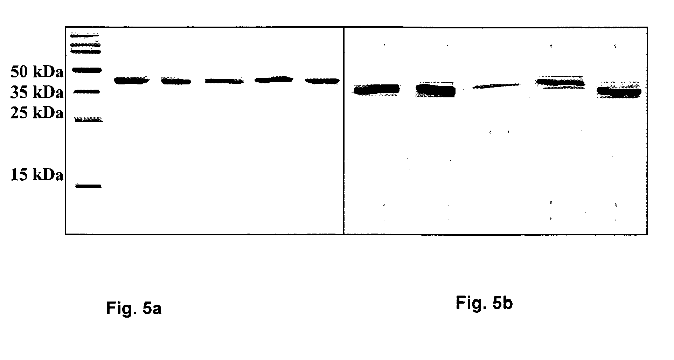

In this study, the recombinant stem bromelain was found to be active and achieved the same characterization as commercial bromelain, so the posttranslational modification may not have affected the protein folding of recombinant bromelain. To confirm this finding, Western blot analysis was carried out with rabbit anti-bromelain acting as the antibody that would bind to the correct band representing bromelain. Fig. 5a shows the SDS-PAGE gel and Fig. 5b is the Western blot analysis from the same sample. The dark band on the membrane is parallel with the results from the gel, suggesting that the heterologous bromelain is isolated, purified and the molecular weight is in the range of 35-50 kDa.

### Stem bromelain gene up-regulation

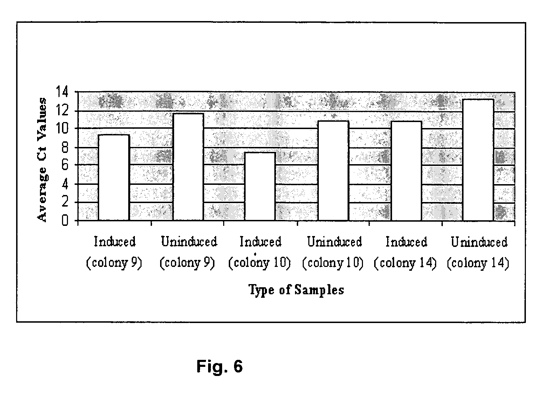

To confirm the recombinant stem bromelain expression from the above finding and to observe the level of expression by L-arabinose induction towards recombinant *E*. *coli* harbouring heterologous bromelain, real-time PCR experiment was carried out. From the results (Fig. 6), the average Ct (cycle threshold) values for the three different colonies are lower for the L-arabinose induced populations compared with the populations without L-arabinose induction. Since the value of Ct is inversely proportional to total RNA, the lower amount of Ct values indicated higher amount of RNA in the samples. The ratio for the protein expression ranged from 0.9 to 1.2 (Table 3) and of these three colonies; colony 10 showed the highest number of expressions compared to colony 9 and colony 14 which the gene up regulated to 1.282 fold. Therefore, RT-PCR has confirmed that the gene was properly transcribed to produce the specific recombinant protein.

**Table 3: Expression comparison for different colonies in real time PCR**

| **Colonies** | **Ratio target gene in induced / control** | **Gene up-regulation** |
|---|---|---|
| 9 | 0.987 | 0.987 fold |
| 10 | 1.282 | 1.282 fold |
| 14 | 0.979 | 0.979 fold |

### Activity of recombinant stem bromelain

Success in cloning and expression cannot confirm that the recombinant stem protein produced is in active form, because problems are often encountered in heterologous gene expression, for example due to misfolding and aggregation of protein in inclusion bodies (Buchner and Mattes, 2005). To determine whether the obtained recombinant stem protein was in active form, two experiments were carried out: a gelatin-plate assay and an enzyme assay.

For the gelatin-plate assay, supernatant collected from recombinant *E*. *coli,* non-recombinant *E*. *coli* (Table 4) were lysed and added into the prepared well. Results have shown that the non-recombinant *E*. *coli* did not demonstrate any halo zone forming, while the recombinant *E*. *coli* supernatant formed a halo zone with a 9 mm diameter. This implied that proteases produced by recombinant *E*. *coli* are not the host proteases but heterologous proteases. It is also suggested that the heterologous bromelain is not an inclusion body because the formation of inclusion bodies is a special mechanism to protect heterologous protein against proteolytic degradation by host proteases (Lilie *et al.* 1998).

**Table 4: Diameter of halo zones measured around the well for different test cultures**

| **Microorganism** | **Diameter of halo zones (mm)** | | | |
|---|---|---|---|---|
| | **Plate 1** | **Plate 2** | **Plate 3** | **Average** |
| Non recombinant *E. coli* | - | - | - | - |
| Recombinant *E. coli* | 8 | 10 | 9 | 9 |

Moreover, the BL21-Al^{™} strain is an *E*. *coli* strain that does not contain the *Ion* protease and is deficient in the outer membrane protease, OmpT, which reduces degradation of heterologous proteins expressed in the strain (Invitrogen user manual).

To measure the enzymic activity of recombinant bromelain, a continuous bromelain assay was done and the characterization of bromelain activity was studied under different temperatures and pH. Recombinant stem bromelain activity assay was performed at 25°C using Nα-CBZ-L-Lysine *p*-nitrophenyl ester, LNPE (50 mM, final concentration), as substrate. One unit of enzyme activity is the amount bromelain that will release 1.0 µmole of *p*-nitrophenol from LNPE per minute under the experimental conditions. The effects of pH and temperature on the activity of the purified recombinant bromelain were investigated.

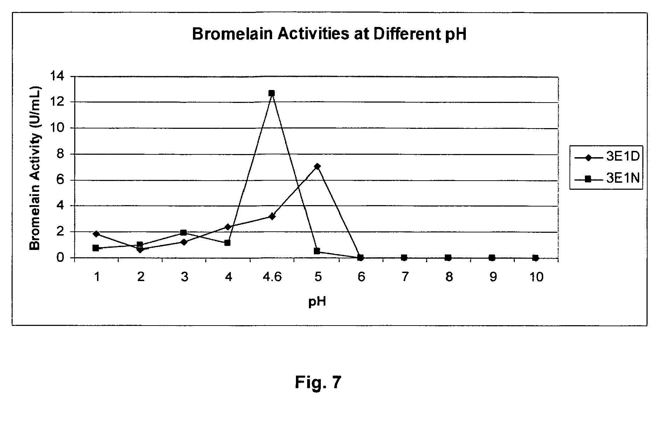

From the pH graph (Fig. 7), the values of bromelain activity for two samples, purified under denatured and native conditions, were higher at pH 4.6 compared to the other pH ranging from 1 to 10. The activity of bromelain increased for native protein from pH 1 to 3 and decreased at pH 4. The value increased tremendously at pH 4.6 and decreased back at pH 5. The denatured and native proteins are inactive at pH 6 to 10. The activity for the denatured protein is not stable decreasing from pH 1 to 2 and continually increasing at pH 3 until 5.

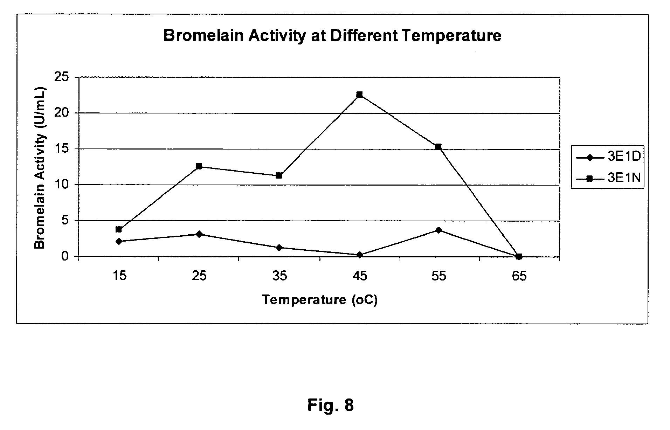

From the temperature graph (Figure 8), the value of bromelain activity for the sample purified under denatured conditions was higher at 55°C compared to native conditions when the activity was higher at 45°C. The activity for the native protein is higher than the denatured protein for all ranges of temperature. The activity of native protein continually increased from 15 to 25°C, fluctuated at 35°C, then increased back and reached its highest point at 45°C. The flow of activity for denatured protein showed a similar pattern to the native protein from 15 to 35°C, but the activity became lowest at 45°C and increased tremendously at 55°C. The activity for this recombinant bromelain was lost at 65°C. Therefore, these results suggested that the recombinant bromelain is active at most of the observed pH and temperature conditions. This is proved by the previous study, the commercial bromelain activity was effective at 40-60°C and deactivate at temperature above 65°C while the optimal pH for the bromelain activity at 4.5 to 5.5 (Heinicke and Gortner, 1957).

### Conclusion

In conclusion, purified recombinant bromelain was detected by Western blot, and the purified enzyme showed hydrolytic activity towards gelatin and a synthetic substrate, Nα-CBZ-L-Lysine *p*-nitrophenyl ester (LNPE). With the latter substrate, the purified recombinant bromelain exhibits optimum activity at pH 4.6 and 45°C.

### References

- M. Rojek, Enzyme Nutrition Therapy, Nexus Magazine, Volume 11, Number 2 (2003).
- T. Harrach et al., Isolation and Partial Characterization of Basic Proteinases from Stem Bromelain, Journal of Protein Chemistry, Vol. 14, No. 1 (1995).
- B. Ravindra Babu, N.K. Rastogi, K.S.M.S. Raghavarao, Liquid-liquid extraction of bromelain and polyphenol oxidase using aqueous two-phase system, Chemical Engineering and Processing 47 (2008) 83-89.
- R.V. Devakate, V.V. Patil, S.S. Waje, B.N. Thorat, Purification and drying of bromelain, Separation and Purification Technology 64 (2009) 259-264.
- J. A. Buchner, R. Mattes, Eschericia coli, In Production of recombinant protein, G. Gellissen (Ed), Wiley-VCH Verlag GmbH & Co. KGaA, Weinheim (2005). Pgs 7-37.
- H. Lilie, E. Schwarz, R. Rudolph, Advance in refolding of protein produces in E. coli, Curr Opin Biotechnol 9 (1998) 497-501.
- Invitrogen user manual, E. coli expression system with Gateway® Technology, (2008), Invitrogen, USA.
- R.M. Heinicke, W.A. Gortner. Stem bromelain-a new protease preparation from pineapple plants. Econ. Bot. 1957.11 (3): 225-234.

## Claims

1. A method for producing recombinant stem bromelain comprising the steps of:
firstly, isolating total RNA from pineapple plant material (11);
then, performing reverse transcription polymerase chain reaction on the RNA to generate multiple copies of stem bromelain gene (12);
then, cloning the stem bromelain gene into cloning vectors (13);
then, transforming the cloning vector into a competent cell to produce an entry clone (14);
then, identifying and selecting the positively transformed entry clone (15);
then, subcloning the stem bromelain gene from the entry clone into a destination vector (16);
then, transforming the destination vector into a host cell to produce recombinant cells (17);
then, identifying and selecting the positively transformed recombinant cells (18);
then, inducing the recombinant cells to express recombinant stem bromelain (19); and
subsequently, harvesting the recombinant cells and purifying the recombinant stem bromelain (20);
wherein the recombinant stem bromelain produced is in an active form and demonstrates activity similar to native stem bromelain.

2. A method for producing recombinant stem bromelain in accordance with claim 1, wherein isolating total RNA from pineapple plant material (11) involves isolating total RNA from the stems of the pineapple plant.

3. A method for producing recombinant stem bromelain in accordance with claim 1, wherein performing reverse transcription polymerase chain reaction on the RNA to generate multiple copies of stem bromelain gene (12) comprises the steps of:
firstly, reverse transcribing the RNA to cDNA using a forward primer BroM-F (5'-AT GGC TTC CAA AGT TC-3') shown in SEQ ID NO. 3 and a reverse primer BroM-R selected from a group consisting of a sequence (5'- CTA AAT CAT TTG CTT CCG ACT T -3') shown in SEQ ID NO. 4, and a sequence (5'- CTA AAT CAT TTG CTT CGA CTT -3') shown in SEQ ID NO. 5;
then, amplifying the cDNA by polymerase chain reaction, to generate multiple copies of the stem bromelain gene;
wherein the forward primer BroM-F may further include additional base pair sequences at the 5' end to ensure compatibility of the BroM-F primer with the cloning vector.

4. A method for producing recombinant stem bromelain in accordance with claim 1, wherein performing reverse transcription polymerase chain reaction on the RNA to generate multiple copies of stem bromelain gene (12) further comprises the step of sequencing the copies of the stem bromelain gene generated by the reverse transcription polymerase chain reaction and blasting said stem bromelain gene against a gene database to confirm the identity of said stem bromelain gene.

5. A method for producing recombinant stem bromelain in accordance with claim 1, wherein cloning the stem bromelain gene into cloning vectors (13) comprises of incubating the stem bromelain gene with reagents comprising salt solution, sterile water and the cloning vector.

6. A method for producing recombinant stem bromelain in accordance with claim 1, wherein the cloning vector is a plasmid.

7. A method for producing recombinant stem bromelain in accordance with claim 1, wherein transforming the cloning vector into a competent cell to produce an entry clone (14) comprises of incubating the cloning vector with a naturally or artificially competent bacterial cell until the stem bromelain gene carried by the cloning vector has transfected the competent cell and integrated with the genome of the competent cell.

8. A method for producing recombinant stem bromelain in accordance with claim 1, wherein the competent cell is *Escherichia coli,* including variants, strains and recombinants thereof.

9. A method for producing recombinant stem bromelain in accordance with claim 1, wherein identification and selection of the positively transformed entry clone (15) is by colony polymerase chain reaction and restriction enzyme digestion by *Cla*I and *Not*I*.*

10. A method for producing recombinant stem bromelain in accordance with claim 1, wherein subcloning the stem bromelain gene from the entry clone into a destination vector (16) is by LR recombination.

11. A method for producing recombinant stem bromelain in accordance with claim 1, wherein the destination vector is a plasmid that can express the stem bromelain gene in a host cell.

12. A method for producing recombinant stem bromelain in accordance with claim 1, wherein transforming the destination vector into a host cell to produce recombinant cells (17) comprises of incubating the destination vector with a naturally or artificially competent bacterial host cell until the stem bromelain gene carried by the destination vector has transfected the host cell and integrated with the genome of the host cell.

13. A method for producing recombinant stem bromelain in accordance with claim 1, wherein the host cell is *Escherichia coli,* including variants, strains and recombinants thereof.

14. A method for producing recombinant stem bromelain in accordance with claim 1, wherein identification and selection of the positively transformed recombinant cells (18) is by colony polymerase chain reaction and restriction enzyme digestion by *Aat*II and *Spe*I.

15. A method for producing recombinant stem bromelain in accordance with claim 1, wherein inducing the recombinant cells to express recombinant stem bromelain (19) comprises the steps of:
firstly, subculturing the positively transformed recombinant cells in a cell culture medium until the mid-logarithmic phase of cell growth;
then, adding L-arabinose to the recombinant cells to induce expression of the recombinant stem bromelain; and
subsequently, incubating the recombinant cells with L-arabinose.

16. A method for producing recombinant stem bromelain in accordance with claim 1, wherein harvesting the recombinant cells and purifying the recombinant stem bromelain (20) comprises the steps of:
firstly, harvesting the recombinant cells;
then, centrifuging the recombinant cells;
then, lysing the recombinant cells to produce a lysate;
then, centrifuging the lysate to collect a supernatant;
and subsequently, extracting the recombinant stem bromelain from the supernatant.
